(19) Europäisches Patentamt
European Patent Office
Office européen des brevets

(11) Publication number: **0 247 832 B2**

(12) # NEW EUROPEAN PATENT SPECIFICATION

(45) Date of publication of the new patent specification: **14.06.95**

(51) Int. Cl.[6]: **C11D 1/94**, C11D 1/65, C11D 1/62, A61K 7/08

(21) Application number: **87304654.4**

(22) Date of filing: **26.05.87**

(54) **Detergent compositions.**

(30) Priority: **27.05.86 JP 121962/86**

(43) Date of publication of application: **02.12.87 Bulletin 87/49**

(45) Publication of the grant of the patent: **02.01.91 Bulletin 91/01**

(45) Mention of the opposition decision: **14.06.95 Bulletin 95/24**

(84) Designated Contracting States: **DE FR GB IT**

(56) References cited:
**EP-A- 0 151 936**
**DE-A- 2 641 058**
**FR-A- 2 233 981**
**US-A- 2 950 255**
**US-A- 4 264 457**

**Product Information Sheet "Sandopan LS-24 Paste" by Sandoz AG (October 1982)**

**Aerztliche Kosmetologie 13, 39-45(83)**

(73) Proprietor: **SHISEIDO COMPANY LIMITED**
**5-5 Ginza 7-chome**
**Chuo-ku**
**Tokyo (JP)**

(72) Inventor: **Nakama, Yasunari c/o Shiseido Laboratories**
**1050, Nippa-cho**
**Kohoku-ku**
**Yokohama-shi**
**Kanagawa (JP)**
Inventor: **Ohkoshi, Masahiro c/o Shiseido Laboratories**
**1050, Nippa-cho**
**Kohoku-ku**
**Yokohama-shi**
**Kanagawa (JP)**

(74) Representative: **Ford, Michael Frederick et al**
**MEWBURN ELLIS**
**York House**
**23 Kingsway**
**London WC2B 6HP (GB)**

EP 0 247 832 B2

Seifen-Oele-Fette-Wachse 108, 12 (1982), pp. 373-376

H. Janistyn, "Taschenbuch der modernen Parfümerie und Kosmetik", (1974), p. 693

Dr. G. Gawalek, "Tenside", (1975), pp. 8,9,118,119,144-147

J. Falbe and U. Hasserodt, "Katalysatoren, Tenside und Mineralöladditive", (1978), pp. 132,134

## Description

The present invention relates to a detergent composition. More specifically, it relates to a detergent composition suitable for use as, for example, a hair or body shampoo or a garment or dish detergent containing a quaternary ammonium cationic surfactant and a carboxylate (i.e., a carboxylic acid salt) anionic surfactant.

Conventionally, anionic surfactants and amphoteric surfactants are generally incorporated, as a main foaming detergent, into shampoos. However, when only these surfactants are incorporated into shampoos, although a sufficient detergent power for washing the hair can be obtained, the applicability of the shampoos is not satisfactory, especially when a so-called "squeak" phenomenon in the hair is remarkable. Various attempts have been made to solve this "squeak" problem by formulating, for example, cationic polymers or cationic surfactants, whereby conditioning effects affording, for example, a smoothness to the hair is intended. However, the use of cationic polymers causes an accumulation thereof on the hair. On the other hand, the amount of cationic surfactants used in shampoos is limited, since cationic surfactants irritate the skin. Accordingly, shampoos having satisfactory conditioning effects have not been obtained, although products having high conditioning effects are needed.

Accordingly, It is desirable to eliminate the above-mentioned disadvantages of the prior art and to provide a detergent composition having an excellent conditioning effect and low skin irritation.

US-A-2950255 discloses a detergent composition comprising in an aqueous medium : (i) at least one amphoteric surfactant ; (ii) at least one quaternary ammonium salt type cationic surfactant ; and (iii) at least one anionic surfactant.

In accordance with the present invention, there is provided a detergent composition of this general type but characterised in that the cationic surfactant (ii) has the formula :

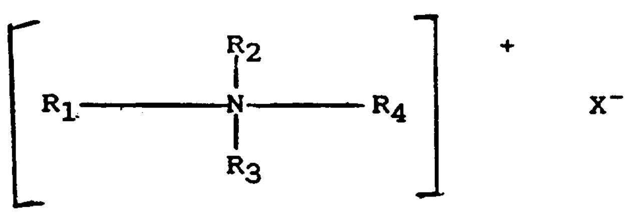

...(I)

wherein $R_1$ represents an alkyl or alkenyl having 12 to 22 carbon atoms, $R_2$, $R_3$, and $R_4$ independently represent methyl or ethyl, and X represents a halogen atom or a methylsulfate residue, and said at least one anionic surfactant is of carboxylate type, the mole ratio of the components (ii)/(iii) being within the range from 4/6 to 8/2 ;

and the total amount of the components (ii) and (iii) is 0.5% to 20% by weight, based on the total amount of the detergent composition.

The present inventors have made an intensive study of a detergent composition such as a shampoo having a conditioning effect and low skin irritation and, as a result, found that the use of monoalkyl quaternary ammonium cationic surfactants and carboxylate anionic surfactants at a specified ratio and in combination with an ampholytic surfactant, can provide a detergent composition satisfying the above-mentioned objects.

When anionic surfactants and cationic surfactants are present together in an aqueous solution, complexes are generally formed in the crystalline phase and the complex is precipitated to make the solution turbid or cause separation of the complex. That is, when quaternary ammonium salt type cationic surfactants are mixed in an aqueous solution with anionic surfactants such as sulfonate type surfactants (e.g., higher alkyl sulfonates), sulfuric acid ester salt type surfactants (e.g., salts of higher alcohol surfates), or phosphoric acid ester salt type surfactants (e.g., salts of polyoxyethylene alkylether phosphonic acid ester), complexes are formed in the crystalline phases at any mole ratio and the resultant complexes are precipitated. Thus, the aqueous solution becomes turbid or the complexes are separated from the aqueous solution. However, when monoalkyl quaternary ammonium salt type cationic surfactants are combined with carboxylic acid salt type anionic surfactants at specific mole ratios in aqueous solutions, the complexes are not formed in the crystalline phases but in the solution. The resultant complexes are transparent and have excellent conditioning effects and low skin irritation. However, although these complexes have the above-mentioned advantages, the foaming power and detergency thereof are not as high as in a conventional shampoo.According to the present invention, the foaming power and the detergency can be advantageously improved, without impairing the desired conditioning effect and the desired skin safety, by incorporating

amphoteric surfactants into the above-mentioned composition.

The amphoteric surfactants usable in the present invention are those which can be used in conventional shampoos. Typical examples of such amphoteric surfactants are imidazoline type amphoteric surfactants such as sodium 2-undecyl-N,N,N-(hydroxyethylcarboxymethyl)-2-imidazoline, 2-cocoyl-2-imidaxoliniumhydroxid-1-carboxyethyloxy-2-sodium ; betaine type amphoteric surfactants such as 2-heptadecyl-N-carboxymethyl-N-hydroxyethyl imidazolinium betaine, betaine lauryl dimethylamino acetate ; alkyl betaines, amino betaines, amine oxides, sulfobetaines.

These amphoteric surfactants may be used alone or in any mixture thereof. Although there are no critical limitations to the amount of the amphoteric surfactant in the composition, this amount is preferably 0.1% to 20% by weight, especially 5% to 15% by weight, based on the total amount of the composition, in the present detergent composition.

The cationic surfactants usable in the present invention are one or more of monoalkyl type quaternary ammonium salts having the above-mentioned general formula (I). When dialkyl type quaternary ammonium cationic surfactants or other cationic surfactans are used, instead of the monoalkyl type, crystallines are precipitated and the desired composition cannot be obtained.

Examples of the carboxylate type anionic surfactants usable in the present invention are as follows :

(A) Fatty acid soap type anionic surfactants having the formula.

$R_6COOM_1$

wherein $R_6$ represents an alkyl or akenyl group having 8 to 18 carbon atoms, $M_1$ represents one or more of alkali metals, organic amines, and basic amino acids ;

(B) Ether carboxylate type anionic surfactants having the formula :

$R_6(OCH_2CH_2)_nOCH_2COOM_2$

wherein $R_6$ represents an alkyl or alkenyl group having 8 to 22 carbon atoms, n is zero or an integer of 1 to 16, and $M_2$ represents one or more of alkali metals, organic amines, and basic amino acids ;

(C) N-acylsarcosinate type anionic surfactants having the formula :

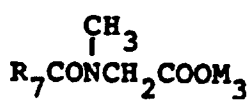

wherein $R_7$ represents an alkyl or alkenyl group having 8 to 18 carbon atoms and $M_3$ represents one or more of alkali metals, organic amines and basic amino acids ; or

(D) Anionic surfactants having a -COO⁻ group in the general formula such as the condensate products of higher fatty acids and amino acids, e.g., N-acylglutamates represented by

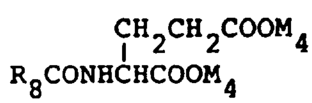

wherein $R_6$ represents an alkyl or alkenyl group having 8 to 18 carbon atoms and $M_4$ independently represents one or more of alkali metals, organic amines, and basic amino acids.

The above-mentioned anionic surfactants may be used alone or in any mixture thereof. The present detergent composition must contain the component (ii), i.e., the quaternary ammonium salt type cationic surfactants, and the component (iii), i.e., carboxylate type anionic surfactants, at a mole ratio of the components (ii)/(iii) of 4/6 to 8/2, preferably 5/5 to 7/3. When the mole ratio is less than 4/6, the conditioning effect of the resultant detergent composition is reduced. Contrary to this, when the mole ratio is more than 8/2, the skin irritation caused by the resultant detergent composition is unpreferably strong.

The total amount of the components (ii) and (iii) in the detergent composition is 0.5% to 20% by weight, preferably 1% to 15% by weight, based on the total amount of the present detergent composition. When the total amount of the components (ii) and (iii) is less than 0.5% by weight, the conditioning effect tends to be decreased because the amount of the surfactants is insufficient. Contrary to this, when the total amount

of the components (ii) and (iii) is more than 20% by weight, the skin irritation tends to become strong.

The detergent compositions according to the present invention may contain, in addition to the above-mentioned essential constituents, any conventional ingredients and additives generally used, as optional components, in detergent compositions, as long as the desired properties of the present invention are not adversely affected. Examples of such optional components are oils such as higher alcohols, silicone oils, liquid paraffins, and ester oils; water-soluble polymers such as methyl cellulose, hydroxyethyl cellulose ; cationic polymers such as cationic modified cellulose ether derivatives (e.g., Polymer JR commercially available from Union Carbide Corporation). The condensate products of polyglycol and polyamine (e.g., polycoat H commercially available from Henkel Corporation) ; nonionic surfactants such as polyoxyethylene (i.e., "POE") hydrogenated castor oil, POE alkylethers ; natural extracts from animals and plants and derivatives thereof ; organic acids such as citric acid and lactic acids; inorganic salts such as sodium chloride and potassium choride ; perfumes or flavors ; coloring agents ; preservatives ; chelating agents ; and UV absorbers.

## Examples

The present invention will now be further illustrated by, but is by no means limited to, the following Examples. The effects were evaluated by the following test and evaluation methods.

## Test and Evaluation Methods

### 1. Conditioning effect

The conditioning effects were evaluated by a panel consisting of 15 women for "smoothness", "glossiness", and "combability" as follows.

A 12 g amount of a sample composition was actually applied to the hair, and after rinsing with lukewarm water, the hair was air dried. The hair was organoleptically evaluated for each item. The evaluation was carried out by the following four grades.

◎ : Very good
o : Good
△ : Fair
x : Poor

### 2. Skin irritation

It is known in the art that skin irritation can be evaluated by a degree of the denaturing activity for proteins (i.e., "protein denaturation") of surfactants or the compositions thereof (see JP-B-59-42038). The skin irritation was evaluated by a protein denaturation in this manner in the following Examples.

The protein denaturation was determined as follows.

A sample composition was added to an egg white albumin solution buffered to a pH of 7 so that the sample concentration was 1%. The denaturing percentage of the egg white albumin was determined by using an absorption peak at 220 nm as follows.

$$\text{Protein denaturation (\%)} = \frac{H_0 - H_S}{H_0} \times 100$$

wherein

$H_0$ : height of absorption peak of egg white albumin at 220 nm

$H_s$ : height of absorption peak at 220 nm when the sample was added to the egg white albumin buffered solution.

The evaluation was carried out by the following four-stage standards.

◎ : Very slight skin irritation (i.e., denaturation is less than 30%)
o : Little skin irritation (i.e., denaturation is not less than 30% but less than 60%)
△ : Middling skin irritation (i.e., denaturation is not less than 60% but less than 80%)
x : Strong skin irritation (i.e., denaturation is 80% or more)

Examples 1 to 7 and Comparative Examples 1 to 7

The detergent compositions having the formulations listed in Table 1 and the rinsing effects and skin irritation thereof were evaluated as mentioned above. The results of Examples 1 to 7 and Comparative Examples 1 to 7 are shown in Table 1.

Table 1

(unit: % by weight)

| Ingredient | Ave. M.W. | Example 1 | Example 2 | Comparative Example 1 | Comparative Example 2 | Comparative Example 3 | Comparative Example 4 | Example 3 | Example 4 | Example 5 | Example 6 | Example 7 | Comparative Example 5 | Comparative Example 6 | Comparative Example 7 |
|---|---|---|---|---|---|---|---|---|---|---|---|---|---|---|---|
| $C_{12}$ alkyltrimethyl ammonium chloride | 264 | 2.04 | - | 2.04 | 2.04 | - | - | - | - | - | - | - | - | - | - |
| $C_{18}$ alkyltrimethyl ammonium chloride | 348 | - | 3.65 | - | - | 1.39 | 2.09 | 2.78 | 3.48 | 4.18 | 4.87 | 5.57 | 6.26 | 0.18 | 18.6 |
| Imidazolium betaine | - | 10 | - | 10 | 10 | - | - | - | - | - | - | - | - | 10 | 10 |
| Betaine lauryldimethylamino acetate | - | - | 15 | - | - | 8 | 8 | 8 | 8 | 8 | 8 | 8 | 8 | - | - |
| Sodium laurate | 222 | 0.75 | - | - | - | - | - | - | - | - | - | - | - | 1.02 | 6.39 |
| Potassium myristoyl glutamate | 433 | - | 1.95 | - | - | 6.93 | 6.06 | 5.20 | 4.33 | 3.46 | 2.60 | 1.73 | 0.87 | - | - |
| Sodium lauryl sulfate | 288 | - | - | 0.87 | - | - | - | - | - | - | - | - | - | - | - |
| Sodium α-olefin sulfonate | 339 | - | - | - | 1.02 | - | - | - | - | - | - | - | - | - | - |
| Water | - | ← Balance → | | | | | | | | | | | | | |
| Mole ratio (Cationic surfactant/Anionic surfactant) | | 7/3 | 7/3 | 7/3 | 7/3 | 2/8 | 3/7 | 4/6 | 5/5 | 6/4 | 7/3 | 8/2 | 9/1 | 0.11/1 | 6.5/3.5 |
| Conditioning effect | | | | | | | | | | | | | | | |
| Smoothness | | ◎ | ◎ | x | x | Δ | o | ◎ | ◎ | ◎ | ◎ | ◎ | o | x | ◎ |
| Glossiness | | ◎ | ◎ | x | x | x | Δ | o | ◎ | ◎ | ◎ | ◎ | o | x | ◎ |
| Combability | | ◎ | ◎ | x | x | x | Δ | ◎ | ◎ | ◎ | ◎ | o | Δ | x | ◎ |
| Skin irritation (protein denaturation) | | ◎ | ◎ | x | x | o | o | ◎ | ◎ | ◎ | ◎ | o | x | o | x |

Example 8

The hair shampoo having the following formulation (i.e., cationic surfactant/anionic surfactant = 6/4 at mole ratio) was prepared and the resultant shampoo was evaluated in the same manner as in Examples 1 to 7.

| Ingredient | % by weight |
|---|---|
| Sodium 2-undecyl-N,N,N-(hydroxyethyl-carboxymethyl)-2-imidazolin | 15.0 |
| Polymer-JR-400 (Union Carbide Corp.) | 0.2 |
| $C_{16}$ alkyltrimethyl ammonium chloride (Ave. molecular weight = 320) | 1.92 |
| Sodium lauroyl alanine (Ave. molecular weight = 293) | 1.23 |
| Propylene glycol | 5.0 |
| Polyoxyethylene (Ave. 40 mole) hydrogenated castor oil derivative | 2.0 |
| Coloring agent | q.s. |
| Perfume | q.s. |
| Deionized water | Balance |

This hair shampoo had excellent conditioning effects and only slight irritation of the skin.

Example 9

The body shampoo having the following formulation (i.e., cationic surfactant/anionic surfactant = 7/3 at mole ratio) was prepared and was evaluated in the same manner as in Examples 1 to 8.

| Ingredient | % by weight |
|---|---|
| Betaine lauryl dimethylamino acetate | 10.0 |
| $C_{22}$ alkenylmethyl ammonium chloride (Ave. molecular weight = 402) | 2.81 |
| Sodium lauroyl sarcosinate (Ave. molecular weight = 293) | 0.93 |
| Dipropylene glycol | 8.0 |
| Aloe extract | 0.5 |
| Coloring agent | q.s. |
| Perfume | q.s. |
| Preservative | q.s. |
| Chelating agent | q.s. |
| Deionized water | Balance |

This body shampoo had excellent conditioning effects and only slight irritation of the skin.

Example 10

The hair shampoo having the following formulation (i.e., cationic surfactant/anionic surfactant = 7/3 at mole ratio) was prepared and the resultant shampoo was evaluated in the same manner as in Examples 1 to 9.

| Ingredient | % by weight |
|---|---|
| Sodium 2-undecyl-N,N,N-(hydroxyethyl-carboxymethyl)-2-imidazolin | 20.0 |
| Polycoat H (Henkel Corp.) | 2.0 |
| Coconut oil fatty acid diethanolamide | 5.0 |
| $C_{16}$ alkyltrimethyl ammonium chloride (Ave. molecular weight = 320) | 2.24 |
| Sodium lauroyl alanine (Ave. molecular weight = 293) | 0.92 |
| Propylene glycol | 3.0 |
| Coloring agent | q.s. |
| Perfume | q.s. |
| Deionized water | Balance |

The hair shampoo had excellent conditioning effects and only slight irritation of the skin.

Example 11

The hair shampoo having the following formulation (i.e., cationic surfactant/anionic surfactant = 6/4 at mole ratio) was prepared and the resultant shampoo was evaluated in the same manner as in Examples 1 to 10.

| Ingredient | % by weight |
|---|---|
| Betain lauryldimethylamino acetate | 20.0 |
| Polycoat-H | 1.0 |
| Coconut fatty acid monoethanolamide | 0.5 |
| $C_{18}$ alkyltrimethyl ammonium chloride (Ave. molecular weight = 348) | 2.09 |
| Sodium lauroylsarcosinate (Ave. molecular weight = 293) | 1.24 |
| Coloring agent | q.s. |
| Perfume | q.s. |
| Deionized water | Balance |

This hair shampoo had excellent conditioning effect and only slight irritation of the skin.

As explained above, according to the present invention, a detergent composition having an excellent conditioning effect and low skin irritation can be provided.

**Claims**

**1.** A hair shampoo composition comprising in an aqueous medium: (1) at least one amphoteric surfactant; (ii) at least one quaternary ammonium salt type cationic surfactant; and (iii) at least one anionic surfactant; characterised in that the cationic surfactant (ii) has the formula

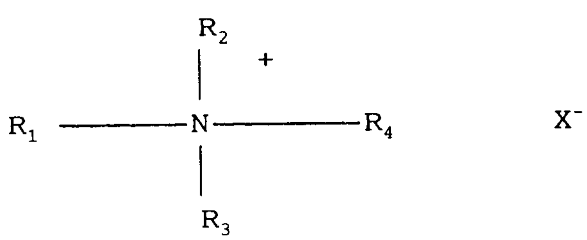 ... (I)

wherein $R_1$ represents an alkyl or alkenyl having 12 to 22 carbon atoms, $R_2$, $R_3$, and $R_4$ independently represent methyl or ethyl, and X represents a halogen atom or a methylsulfate residue, and said at least one anionic surfactant is of carboxylate type selected from

(a) at least one fatty acid soap type anionic surfactant having the formula (II):

$R_5COOM_1$ (II)

wherein $R_5$ represents an alkyl or alkenyl group having 8 to 18 carbon atoms and $M_1$ represents one or more of alkali metals, organic amines, and basic amino acids;

(b) at least one N-acylsarcosinate type anionic surfactant having the formula (III):

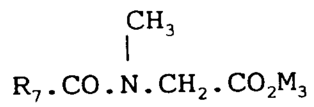

(III)

wherein $R_7$ represent an alkyl or alkenyl group having 8 to 18 carbon atoms and $M_3$ represents one or more of alkali metals, organic amines and basic amino acids; and

(c) at least one N-acylglutamate represented by the formula (IV):

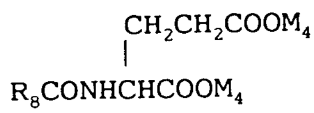

(IV)

wherein $R_8$ represents an alkyl or alkenyl group having 8 to 18 carbon atoms and $M_4$ independently represents one or more of alkali metals, organic amines, and basic amino acids;

the mole ratio of the components (ii)/(iii) being within the range from 4/6 to 8/2;

and the total amount of the components (ii) and (iii) being 0.5% to 20% by weight, based on the total amount of the detergent composition.

**2.** A composition as claimed in claim 1, wherein the amount ofthe amphoteric surfactant is 0.1% to 20% by weight based on the total weight of the composition.

**3.** A composition as claimed in claim 1 or 2 wherein the mole ratio of the components (ii)/(iii) is 5/5 to 7/3.

**4.** A composition according to claim 1, 2 or 3 wherein the anionic surfactant is of formula II.

**5.** A composition according to claim 1, 2 or 3 wherein the anionic surfactant is of formula III.

**6.** A composition according to claim 1, 2, or 3 wheren the anionic surfactant is of formula IV.

**Patentansprüche**

**1.** Haar-Shampoo-Zusammensetzung mit in einem wäßrigen Medium: (i) mindestens einem amphoteren oberflächenaktiven Mittel; (ii) mindestens einem kationischen oberflächenaktiven Mittel vom Typ eines quaternären Ammoniumsalzes; und (iii) mindestens einem anionischen oberflächenaktiven Mittel; dadurch gekennzeichnet, daß das kationische oberflächenaktive Mittel (ii) der folgenden Formel entspricht:

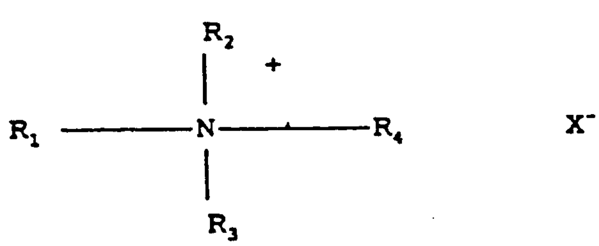

(I)

worin bedeuten: $R_1$ eine Alkyl- oder Alkenylgruppe mit 12 bis 22 Kohlenstoffatomen, $R_2$, $R_3$ und $R_4$ unabhängig voneinander Methyl oder Ethyl und X ein Halogenatom oder einen Methylsulfatrest, und daß mindestens ein anionisches oberflächenaktives Mittel vom Carboxylat-Typ ist, ausgewählt aus:

(a) mindestens einem anionischen oberflächenaktiven Mittel vom Typ einer Fettsäureseife mit der Formel (II):

$R_5COOM_1$ (II)

worin $R_5$ für eine Alkyl- oder Alkenylgruppe mit 8 bis 18 Kohlenstoffatomen steht und $M_1$ eine oder mehrere Alkalimetalle, organische Amine und basische Aminosäuren darstellt;
(b) mindestens einem anionischen oberflächenaktiven Mittel vom N-Acylsarcosinat-Typ mit der Formel (III):

$$R_7.CO.\overset{\displaystyle CH_3}{\overset{|}{N}}.CH_2.CO_2M_3 \quad (III)$$

worin $R_7$ für eine Alkyl- oder Alkenylgruppe mit 8 bis 18 Kohlenstoffatomen steht, und worin $M_3$ ein oder mehrere Alkalimetalle, organische Amine und basische Aminosäuren darstellt; und
(c) mindestens einem N-Acylglutamat, das durch die folgende Formel (IV) dargestellt wird:

$$R_8CONH\overset{\displaystyle CH_2CH_2COOM_4}{\overset{|}{C}}HCOOM_4 \quad (IV)$$

worin $R_8$ für eine Alkyl- oder Alkenylgruppe mit 8 bis 18 Kohlenstoffatomen steht und $M_4$ unabhängig voneinander steht für ein oder mehrere Alkalimetalle, organische Amine und basische Aminosäuren;
wobei das Mol-Verhältnis der Komponenten (ii)/(iii) im Bereich von 4/6 bis 8/2 liegt;
und wobei die Gesamtmenge der Komponenten (ii) und (iii) bei 0,5 bis 20 Gew.-%, bezogen auf die Gesamtmenge an der Waschmittel-Zusammensetzung liegt.

**2.** Zusammensetzung nach Anspruch 1, in der die Menge an dem amphoteren oberflächenaktiven Mittel bei 0,1 bis 20 Gew.-%, bezogen auf das Gesamtgewicht der Zusammensetzung liegt.

**3.** Zusammensetzung nach Anspruch 1 oder 2, in der das Mol-Verhältnis der Komponenten (ii)/(iii) bei 5/5 bis 7/3 liegt.

**4.** Zusammensetzung nach Anspruch 1, 2 oder 3, in der das anionische oberflächenaktive Mittel ein solches der Formel II ist.

**5.** Zusammensetzung nach Anspruch 1, 2 oder 3, in der das anionische oberflächenaktive Mittel ein solches der Formel III ist.

**6.** Zusammensetzung nach Anspruch 1, 2 oder 3, in der das anionische oberflächenaktive Mittel ein solches der Formel IV ist.

## Revendications

**1.** Un shampooing pour cheveux comprenant, dans un milieu aqueux:
(i) au moins un tensioactif amphotère;
(ii) au moins un tensioactif cationique du type sel d'ammonium quaternaire; et
(iii) au moins un tensioactif anionique;
caractérisée en ce que le tensioactif cationique (ii) a la formule suivante:

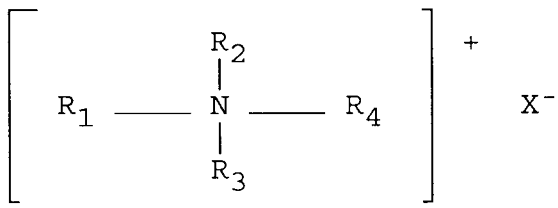

(I)

dans laquelle:

| | |
|---|---|
| $R_1$ | représente un radical allyle ou alcényle ayant de 12 à 22 atomes de carbone; |
| $R_2$, $R_3$ et $R_4$, | indépendamment les uns des autres, représentent chacun le radical méthyle ou éthyle; et |
| X | représente un atome d'halogène ou un résidu méthylsulfate, |

et ledit au moins un tensioactif anionique est du type carboxylate, choisi dans le groupe consistant en

(a) au moins un tensioactif anionique de type savon d'acide gras de formule (II)

$R_5COOM_1$ (II)

dans laquelle $R_5$ représente un groupe alkyle ou alkényle ayant de 8 à 18 atomes de carbone et $M_1$ représente un ou plusieurs métaux alcalins, amines organiques et amino-acides à caratère basique;

(b) au moins un tensioactif anionique de type N-acylsarcosinate de formule (III):

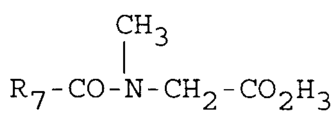

(III)

dans laquelle $R_7$ représente un groupe alkyle ou alkényle ayant de 8 à 18 atomes de carbone et $M_3$représente un ou plusieurs métaux alcalins, amines organiques, et amino-acides à caractère basique; et

(c) au moins un N-acylglutamate de formule (IV)

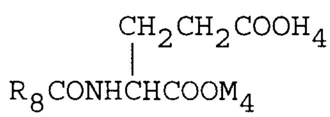

(IV)

dans laquelle $R_8$ représente un groupe alkyle ou alkényle ayant de 8 à 18 atomes de carbone et $M_4$ représente indépendamment un ou plusieurs métaux alcalins, amines organiques, et aminoacides à caractère basique; la proportion molaire des composants (ii)/(iii) étant comprise entre 4/6 et 8/2;

et la quantité totale des composés (ii) et (iii) est de 0,5% à 20% en poids par rapport à la quantité totale de la composition détergente.

**2.** Un shampooing selon la revendication 1, dans laquelle la quantité du tensioactif amphotère est de 0,1% à 20% en poids par rapport au poids total de la composition.

**3.** Un shampooing selon la revendication 1 ou 2, dans laquelle la proportion molaire des composants (ii)/-(iii) est de 5/5 à 7/3.

**4.** Un shampooing selon la revendication 1, 2 ou 3, dans laquelle ledit tensioactif anionique de type carboxylate est au moins un tensioactif anionique est de formule II.

**5.** Un shampooing selon la revendication 1, 2 ou 3, dans laquelle ledit tensioactif anionique de type carboxylate est au moins un tensioactif anionique est de formule III.

**6.** Un shampooing selon la revendication 1, 2 ou 3, dans laquelle ledit tensioactif anionique de type carboxylate est au moins un tensioactif anionique est de formule IV.